# EUROPEAN PATENT SPECIFICATION

(11) **EP 3 437 681 B1**
(45) Date of publication and mention of the grant of the patent: **03.04.2024**
(21) Application number: 17775172.4
(22) Date of filing: 29.03.2017
(51) Int. Cl.: A61M 5/315, A61M 5/00, A61M 5/31

(54) **MEDICINE ADMINISTERING APPARATUS, METHOD OF USING MEDICINE ADMINISTERING APPARATUS, AND METHOD FOR MANUFACTURING MEDICINE ADMINISTERING APPARATUS**
VORRICHTUNG ZUR VERABREICHUNG VON MEDIKAMENTEN, VERFAHREN ZUR VERABREICHUNG VON MEDIKAMENTEN UND VERFAHREN ZUR HERSTELLUNG EINER VORRICHTUNG ZUR VERABREICHUNG VON MEDIKAMENTEN
APPAREIL D'ADMINISTRATION DE MÉDICAMENT, PROCÉDÉ D'UTILISATION D'APPAREIL D'ADMINISTRATION DE MÉDICAMENT ET PROCÉDÉ DE FABRICATION D'APPAREIL D'ADMINISTRATION DE MÉDICAMENT

(30) Priority: 29.03.2016 JP 2016066145
(43) Date of publication of application: 06.02.2019
(73) Proprietor: Terumo Kabushiki Kaisha, Tokyo 151-0072 (JP)
(72) Inventor: OGAWA, Junichi, Nakakoma-gun Yamanashi 409-3853 (JP); KOIWAI, Kazunori, Ashigarakami-gun Kanagawa 259-0151 (JP); URA, Takehiro, Ashigarakami-gun Kanagawa 259-0151 (JP)
(74) Representative: Casalonga
(86) International application number: PCT/JP2017/012795
(87) International publication number: WO 2017/170635

(56) References cited:
- EP-A1- 0 511 183
- EP-A1- 2 962 716
- JP-A- 2003 199 824
- JP-A- 2004 313 369
- US-A- 5 785 682
- US-A1- 2006 211 995

## Description

### Technical Field

The present invention relates to a drug-solution administration device including a syringe that can be filled with a drug solution and a gasket slidably inserted into the syringe, a method of using the drug-solution administration device, and a method of manufacturing the drug-solution administration device.

### Background Art

In general, a drug-solution administration device includes: a syringe that can be filled with a drug solution; a gasket slidably inserted into the syringe; and a plunger member that pushes the gasket (see, for example,

JP 2011-212185A).

In addition, it is necessary to execute sterilization treatment on the syringe and the gasket. In recent years, radiation sterilization such as gamma rays and electron radiation has been performed as a method of the sterilization treatment. Incidentally, there is a risk that the gasket may deteriorate in the radiation sterilization so that there is a demand for a sterilization method using high-temperature steam or gases such as high-pressure steam sterilization (autoclave) and ethylene oxide gas (EOG) sterilization (see, for example, EP 2 962 716 A1)

### Summary of Invention

### Technical Problem

However, the steam or gases for sterilization hardly reaches an inner wall of the syringe in close contact with the gasket and a space surrounded by two peak portions formed in the gasket and the inner wall of the syringe so that there is a risk that the syringe and gasket are not sufficiently sterilized. Thus, conventionally, when sterilizing the syringe using the high-temperature steam or gases, it is necessary to separately sterilize the syringe and the gasket and then to assemble the syringe and the gasket as a drug-solution administration device.

The present invention has been made in view of the above problems, and an object thereof is to provide a drug-solution administration device, a method of using a drug-solution administration device, and a method of manufacturing a drug-solution administration device capable of reliably perform sterilization using steam or gases in a state where a syringe and a gasket are assembled in advance as the drug-solution administration device.

### Solution to Problem

In order to solve the above-described problems and achieve the object of the present invention, a drug-solution administration device according to claim 1 and a method of manufacturing a drug-solution administration device according to claim 8 are provided.

### Advantageous Effects of Invention

According to the drug-solution administration device and the method of manufacturing the drug-solution administration device of the present invention, it is possible to reliably perform sterilization in the state where the syringe and the gasket are assembled in advance as the drug-solution administration device when performing the sterilization treatment using steam or gases.

### Brief Description of Drawings

Fig. 1 is a perspective view illustrating a drug-solution administration device according to an embodiment of the present invention.
Fig. 2 is a cross-sectional view illustrating the drug-solution administration device according to the embodiment of the present invention.
Fig. 3 is an explanatory view illustrating states of a plunger member and a gasket before sterilization treatment in the drug-solution administration device according to the embodiment of the present invention.
Fig. 4 is a perspective view illustrating the plunger member and the gasket of the drug-solution administration device according to the embodiment of the present invention.
Fig. 5 is a perspective view illustrating a temporary holding portion of the drug-solution administration device according to the embodiment of the present invention.
Fig. 6 is an explanatory view illustrating the plunger member and the temporary holding portion before sterilization treatment in the drug-solution administration device according to the embodiment of the present invention.
Fig. 7 is a perspective view illustrating a state at the time of sucking a drug solution in the drug-solution administration device according to the embodiment of the present invention.
Fig. 8 is a cross-sectional view illustrating a state after the sterilization treatment in the drug-solution administration device according to the embodiment of the present invention.
Fig. 9 is a cross-sectional view illustrating a state where the drug solution has been sucked in the drug-solution administration device according to the embodiment of the present invention.
Fig. 10 is a cross-sectional view illustrating a state after administration of the drug solution in the drug-solution administration device according to an embodiment of the present invention.

### Description of Embodiments

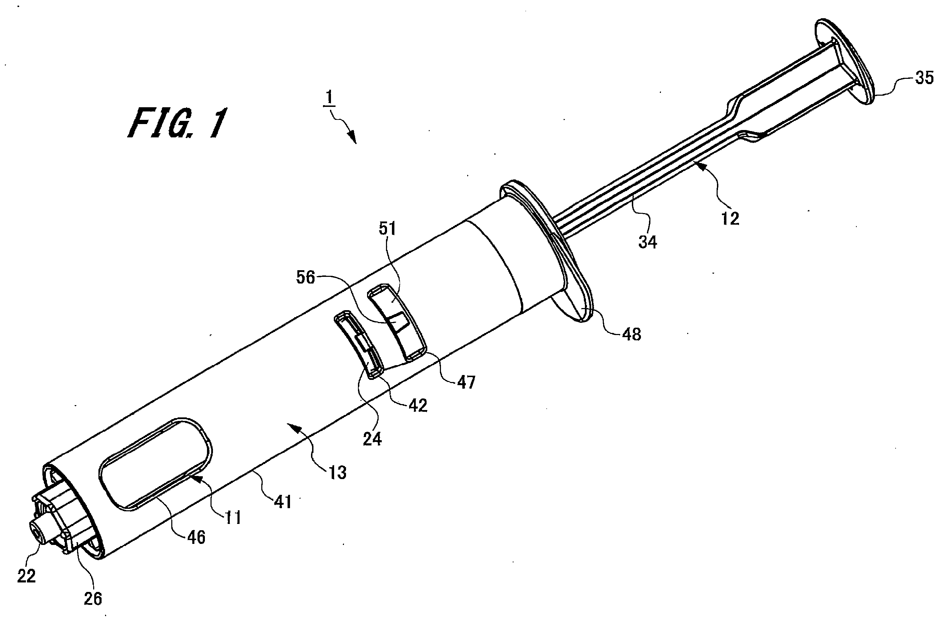

Hereinafter, an embodiment of a drug-solution administration device, a use method (not covered by the claimed invention) and a manufacturing method of the same of the present invention will be described with reference to Figs. 1 to 10. Common members are denoted by identical reference signs throughout the drawings. In addition, the present invention is not limited to the following embodiment.

### 1. Embodiment

### [Configuration of Drug-solution administration device]

First, a configuration of an embodiment (hereinafter referred to as "the present embodiment") of the drug-solution administration device of the present invention will be described with reference to Figs. 1 to 6.

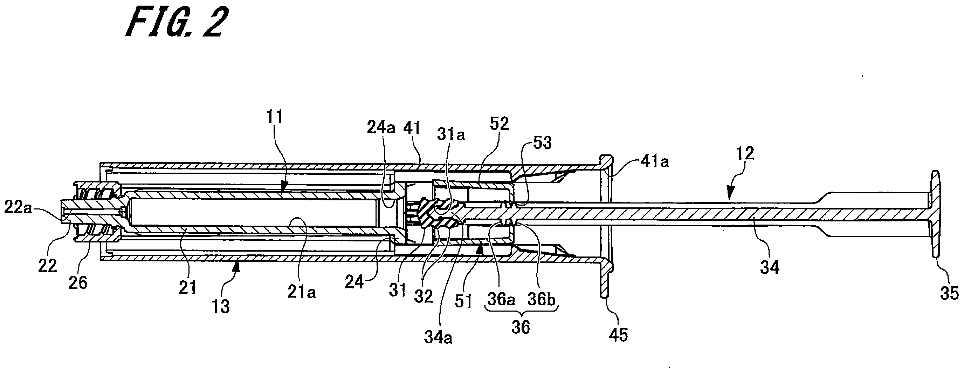

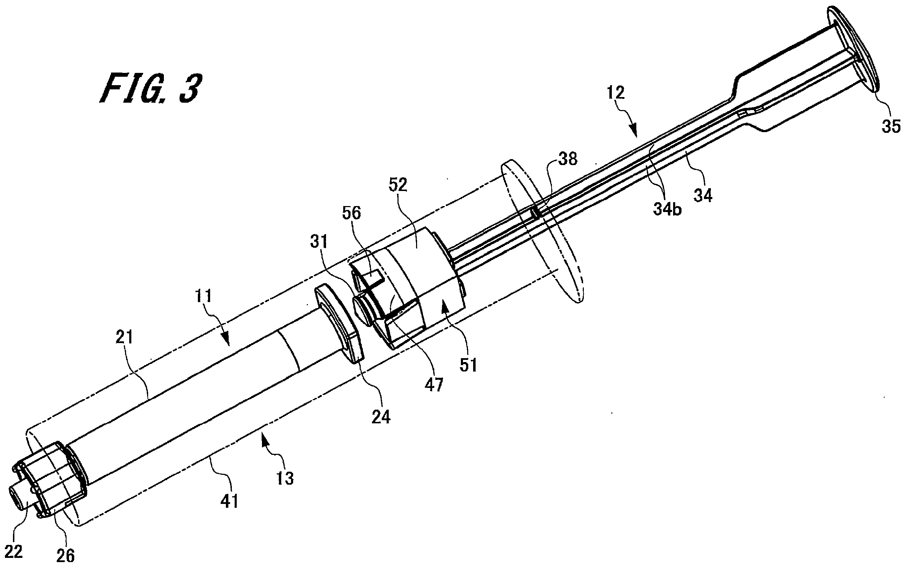

Fig. 1 is a perspective view illustrating the drug-solution administration device according to the present embodiment. Fig. 2 is a cross-sectional view illustrating administration parts of the drug-solution administration device of the present embodiment. Fig. 3 is an explanatory view illustrating states of a plunger member, a gasket, and a syringe of the present embodiment.

As illustrated in Fig. 1, a drug-solution administration device 1 includes a syringe 11, a plunger member 12, a syringe holder 13 that holds the syringe 11, and a temporary holding portion 51 that temporarily holds the plunger member.

### [Syringe]

The syringe 11 is a syringe filled with a drug solution M1 to be described later. The syringe 11 has a body portion 21 formed in a substantially cylindrical shape and a discharge portion 22 formed at a distal end portion of the body portion 21.

A gasket 31 to be described later is slidably inserted into the cylindrical hole 21a of the body portion 21. When the gasket 31 is inserted into the cylindrical hole 21a, a space of the body portion 21 closer to the discharge portion 22 than the gasket 31 and a space inside the discharge portion 22 form a liquid chamber to be filled with the drug solution M1.

Examples of the drug solution M1 may include various vaccines to prevent various infectious diseases, such as influenza, but the drug solution is not limited to the vaccine. Examples of drug solutions M1 other than the vaccine may include a sugar injection solution like glucose, an injection solution for correction of electrolyte, such as sodium chloride or potassium lactate, vitamins, antibiotic infusion solutions, contrast media, steroids, protease inhibitors, fat emulsions, anticancer agents, anesthetic, heparin calcium, antibody preparations, and the like.

Further, a flange portion 24 is provided at a proximal end portion of the body portion 21. The flange portion 24 protrudes radially outward from an outer peripheral surface of the proximal end portion of the body portion 21. The flange portion 24 is engaged with a through-hole 42 provided in the syringe holder 13 to be described later. As a result, the syringe 11 is held by the syringe holder 13.

In addition, an opening 24a communicating with the cylindrical hole 21a of the body portion 21 is formed in the flange portion 24. The opening 24a is open in a circular shape. The opening 24a is opposed to the gasket 31 to be described later in the state before performing the sterilization treatment as illustrated in Figs. 2 and 3. In addition, the gasket 31 and a plunger body 34, which will be described later, are inserted into the opening 24a at the time of use.

The discharge portion 22 is continuous with one end of the body portion 21 and formed in a substantially cylindrical shape coaxial with the body portion 21. The discharge portion 22 is formed in a tapered shape whose diameter continuously decreases toward a distal end opposite to the body portion 21. A cylindrical hole 22a of the discharge portion 22 communicates with the cylindrical hole 21a of the body portion 21.

A luer lock portion 26, which is an example of a screw portion, is joined to the discharge portion 22. A needle hub 82 having a suction needle tube 81 configured to suction a drug solution and a needle hub having a needle tube that can pierce a living body are releasably attached to the luer lock portion 26.

Examples of a material of the syringe 11 may include various types of resin such as polyvinyl chloride, polyethylene, polypropylene, cyclic polyolefin, polystyrene, poly-(4-methylpentene-1), polycarbonate, acrylic resin, an acrylonitrile-butadiene-styrene copolymer, polyester such as polyethylene terephthalate, a butadiene-styrene copolymer, and polyamide (for example, nylon 6, nylon 6/6, nylon 6/10, and nylon 12). Among them, it is preferable to use resin such as polypropylene, cyclic polyolefin, polyester, and poly-(4-methylpentene-1) from the viewpoint that molding is easy. Incidentally, it is preferable that the material of the syringe 11 be substantially transparent in order to secure the visibility of the interior.

### [Plunger Member]

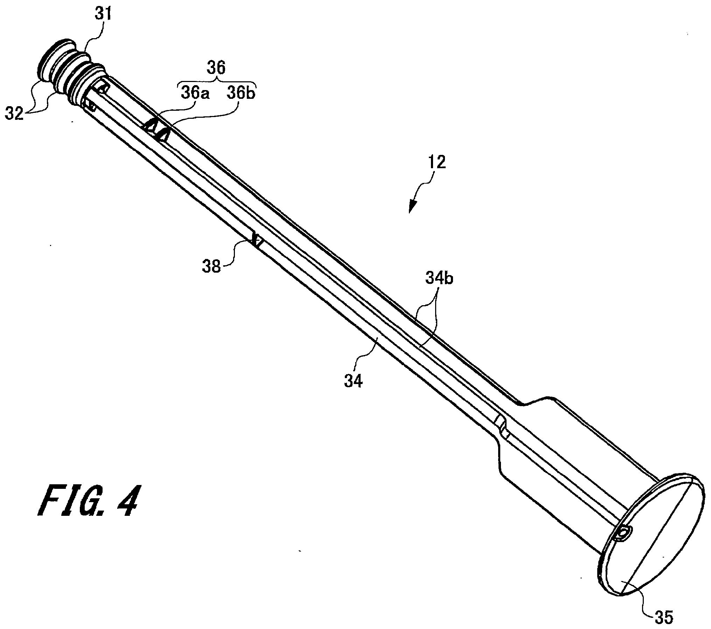

Fig. 4 is a perspective view illustrating the plunger member 12.

As illustrated in Fig. 4, the plunger member 12 includes the gasket 31, the plunger body 34 that presses the gasket 31, an operation portion 35, a plunger-side engaging portion 36, and a plunger-side retaining portion 38.

As illustrated in Fig. 2, the gasket 31 is formed in a substantially columnar shape. One end portion of the gasket 31 is formed in a tapered shape whose diameter continuously decreases toward a distal end. This tapered shape corresponds to a shape of an inner surface at the distal end portion of the body portion 21. Therefore, when the gasket 31 is moved toward the distal end portion of the body portion 21, the one end portion of the gasket 31 contacts the inner surface of the distal end portion of the body portion 21 so as not to generate a gap.

In addition, a connecting hole 31a is formed at the other end portion of the gasket 31 as illustrated in Fig. 2. A connecting portion 34a of the plunger body 34 is inserted into the connecting hole 31a so that the gasket 31 and the plunger body 34 are connected.

Further, two peak portions 32 and 32 are provided on an outer peripheral surface of the gasket 31. The two peak portions 32 and 32 are convex portions protruding in a ring shape from the outer peripheral surface of the gasket 31. The two peak portions 32 and 32 are provided with a gap therebetween in the axial direction of the gasket 31. When the gasket 31 is inserted into the cylindrical hole 21a of the syringe 11, the two peak portions 32 and 32 are brought into liquid-tight contact with an inner wall of the cylindrical hole 21a.

A material of the gasket 31 is not particularly limited, but it is preferable to use an elastic material in order to obtain favorable liquid tightness with the body portion 21. Examples of the elastic material may include various rubber materials, such as natural rubber, isobutylene rubber, butyl rubber, and silicone rubber, various thermoplastic elastomers, such as an olefin type and a styrene type, or mixtures thereof.

The plunger body 34 is formed in a substantially rod shape, and a sectional shape in a direction orthogonal to an axial direction of the plunger body 34 is formed in a substantially cross shape. Further, the plunger body 34 is constituted by four plunger pieces 34b protruding in a direction orthogonal to the axial direction. The four plunger pieces 34b are continuous to be substantially vertical to each other.

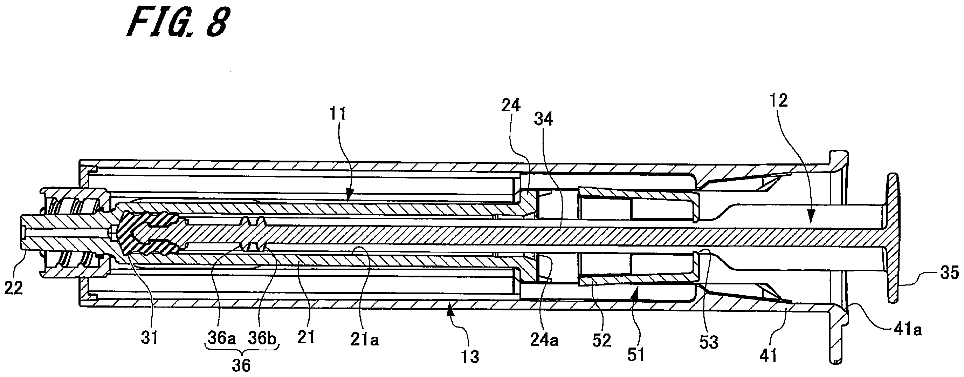

As illustrated in Figs. 2 and 3, the plunger body 34 is opposed to the opening 24a of the syringe 11 in the state of being held by the temporary holding portion 51 to be described later in the state before performing the sterilization treatment. After performing the sterilization treatment, the plunger body 34 is inserted into the body portion 21 from the opening 24a of the syringe 11 at the time of use, and the most thereof is arranged inside the body portion 21 of the syringe 11 (see Fig. 8).

In addition, the connecting portion 34a is provided at the distal end portion of the plunger body 34. The connecting portion 34a is inserted into the connecting hole 31a of the gasket 31 and is connected to the gasket 31. As a result, the gasket 31 is attached to the distal end portion of the plunger body 34.

The operation portion 35 is provided at a proximal end portion of the plunger body 34. The operation portion 35 is formed in a substantially disc shape. When the operation portion 35 is pressed by the user at the time of using the drug-solution administration device 1, the gasket 31 arranged at the distal end of the plunger body 34 moves inside the cylindrical hole 21a of the body portion 21 of the syringe 11.

In addition, the plunger-side engaging portion 36 and the plunger-side retaining portion 38 are provided on a shaft portion of the plunger body 34. The plunger-side engaging portion 36 is provided on a distal end side in the axial direction of the plunger body 34. The plunger-side engaging portion 36 is provided between two plunger pieces 34b and 34b adjacent to each other among the four plunger pieces 34b of the plunger body 34 formed in the cross shape. Further, the plunger-side engaging portion 36 is provided at two places symmetric with each other in a direction orthogonal to the axial direction of the plunger body 34 with an axial center of the plunger body 34 interposed between the two places.

In addition, the plunger-side engaging portion 36 has a first engaging portion 36a and a second engaging portion 36b. The first engaging portion 36a and the second engaging portion 36b are arranged with a predetermined gap therebetween in the axial direction of the plunger body 34. The first engaging portion 36a is arranged to be closer to the distal end side in the axial direction of the plunger body 34 than the second engaging portion 36b.

The plunger-side retaining portion 38 is provided to be closer to a proximal end side in the axial direction of the plunger body 34 than the plunger-side engaging portion 36. The plunger-side retaining portion 38 is formed between two plunger pieces 34b and 34b. which are different from a space between the two plunger pieces 34b and 34b where the plunger-side engaging portion 36 is provided among the four plunger pieces 34b of the plunger body 34.

### [Syringe Holder]

As illustrated in Figs. 1 and 2, the syringe holder 13 has a barrel body portion 41, a through-hole 42, a viewing window 46, a locking hole 47, and a holder collar 48.

The barrel body portion 41 is formed in a substantially cylindrical shape and covers outer peripheral surfaces of the body portion 21 and the flange portion 24 of the syringe 11 and an outer peripheral surface of the luer lock portion 26. The barrel body portion 41 is configured to be grippable by a user. A holder opening 41a into which the syringe 11 and the plunger member 12 can be inserted is formed at a proximal end portion of the barrel body portion 41.

The holder opening 41a is positioned to be closer to the distal end side, which is the proximal end portion of the plunger body 34 inserted into the syringe holder 13, than the operation portion 35. Incidentally, it suffices that the barrel body portion 41 covers at least the outer peripheral surface of the flange portion 24.

The viewing window 46 is opened at a distal end portion of the barrel body portion 41. The viewing window 46 is provided at a position where the liquid chamber formed in the body portion 21 of the syringe 11 is visible from the outside of the syringe holder 13 when the syringe 11 is attached to the syringe holder 13. Accordingly, it is possible to secure the visibility of the interior even if the syringe holder 13 is attached to the syringe 11.

Further, the holder collar 48 is provided at the proximal end portion of the barrel body portion 41. The holder collar 48 protrudes to be substantially vertical from a part of the outer peripheral surface of the barrel body portion 41. Since the holder collar 48 is provided, it is possible to prevent a finger gripping the syringe holder 13 from slipping toward the proximal end direction when the user grips the syringe holder 13 and administers the drug solution. In addition, it is also possible to prevent the drug-solution administration device 1 from rolling when placing the drug-solution administration device 1 on a desk or the like.

In addition, when the syringe 11 and the plunger member 12 are inserted into the barrel body portion 41 from the holder opening 41a, it is possible to determine a direction in which the syringe 11 and the plunger member 12 are inserted in accordance with a short-diameter portion of the holder collar 48.

In addition, the through-hole 42 and the locking hole 47 are opened in the barrel body portion 41. The through-hole 42 and the locking hole 47 are formed to be closer to the proximal end portion side in the axial direction of the barrel body portion 41 than the viewing window 46. The flange portion 24 of the syringe 11 is locked by the through-hole 42. As a result, the syringe 11 is held inside the barrel body portion 41.

The locking hole 47 is formed in the vicinity of the through-hole 42 to be closer to the proximal end portion side in the axial direction of the barrel body portion 41 than the through-hole 42. Thus, the locking hole 47 is formed to be closer to the proximal end side in the axial direction of the barrel body portion 41 than the flange portion 24 of the syringe 11 held by the barrel body portion 41. A locking portion of the temporary holding portion 51 is locked by the locking hole 47.

### [Temporary Holding Portion]

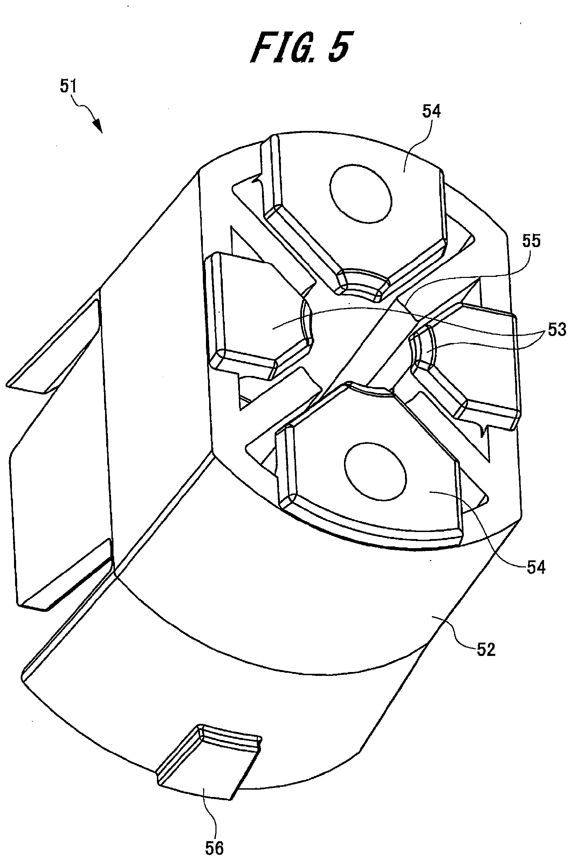

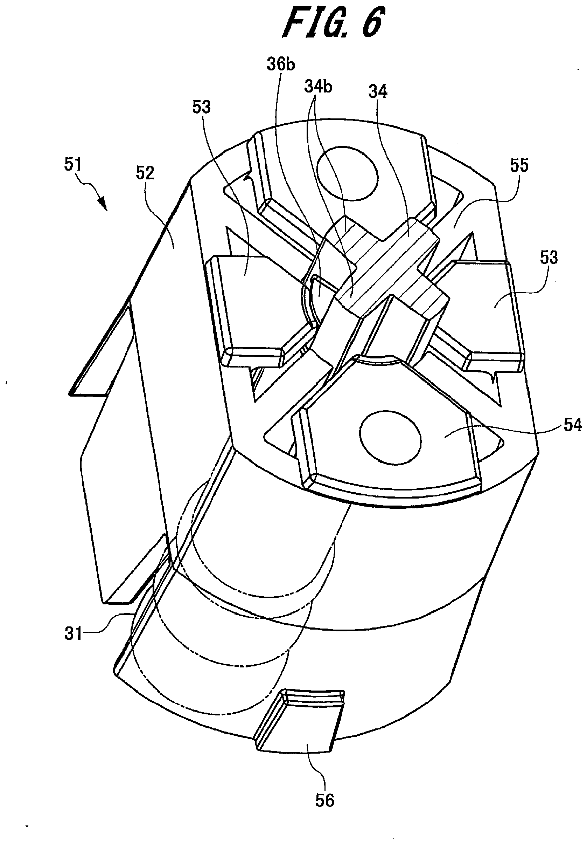

Next, the temporary holding portion 51 will be described with reference to Figs. 5 and 6.

Fig. 5 is a perspective view illustrating the temporary holding portion 51. Fig. 6 is a perspective view illustrating the temporary holding portion 51 and the plunger member 12.

As illustrated in Fig. 5, the temporary holding portion 51 includes a cylindrical body 52, two holding-portion-side engaging portions 53 and 53, two holding-portion-side retaining portions 54 and 54, a plurality of support ribs 55, and a locking portion 56. The cylindrical body 52 is formed in a cylindrical shape. One axial end of the cylindrical body 52 is open. In addition, the two holding-portion-side engaging portions 53 and 53 and two holding-portion-side retaining portions 54 and 54 are provided on the other axial end portion of the cylindrical body 52.

The two holding-portion-side engaging portions 53 and 53 are arranged at positions opposed to each other at an edge portion of an opening formed at the other end portion of the cylindrical body 52. The two holding-portion-side engaging portions 53 and 53 are bent to be substantially vertical from the edge portion of the cylindrical body 52 toward the inside of a cylindrical hole of the cylindrical body 52.

As illustrated in Figs. 2 and 6, the holding-portion-side engaging portion 53 is releasably engaged with the plunger-side engaging portion 36 provided on the plunger body 34. That is, the holding-portion-side engaging portion 53 is arranged between the first engaging portion 36a and the second engaging portion 36b of the plunger-side engaging portion 36. Then, the first engaging portion 36a is engaged with the holding-portion-side engaging portion 53 from the distal end side, and the second engaging portion 36b is engaged with the holding-portion-side engaging portion 53 from the proximal end side Match. As a result, the plunger member 12 is temporarily held by the temporary holding portion 51 in a releasable manner.

The two holding-portion-side retaining portions 54 and 54 illustrating one example of a retaining portion are formed at the edge portion of the opening formed at the other end portion of the cylindrical body 52 between the two holding-portion-side engaging portions 53 and 53. In addition, the two holding-portion-side retaining portions 54 and 54 are arranged at positions opposed to each other. The two holding-portion-side retaining portions 54 and 54 are bent to be substantially vertical from the edge portion of the cylindrical body 52 toward the inside of the cylindrical hole of the cylindrical body 52. Then, insertion holes through which the plunger body 34 is inserted are formed by distal end portions of the two holding-portion-side engaging portions 53 and 53 and the two holding-portion-side retaining portions 54 and 54.

Although the example in which the retaining portion is provided in the temporary holding portion 51 has been described in this example, the present invention is not limited thereto, and the retaining portion and the temporary holding portion 51 may be formed using different members.

The plurality of support ribs 55 (four in the present embodiment) illustrating one example of a support portion are formed between the holding-portion-side engaging portion 53 and the holding-portion-side retaining portion 54. The plurality of support ribs 55 protrude from an inner wall of the cylindrical body 52 toward the inside of the cylindrical hole with a predetermined length. In addition, the plurality of support ribs 55 extend from the other end portion of the cylindrical body 52 to a midway portion in the axial direction.

As illustrated in Fig. 6, when the plunger body 34 is inserted into the temporary holding portion 51, the plurality of support ribs 55 contacts the plunger piece 34b of the plunger body 34. Therefore, the plunger body 34 is held by the plurality of support ribs 55. Thereby, the plunger member 12 can be held by the temporary holding portion 51 without rattling. In addition, the gasket 31 is covered by the cylindrical body 52 of the temporary holding portion 51 in the state before performing sterilization treatment as illustrated in Figs. 3 and 6. As a result, it is possible to prevent the user's finger from contacting the gasket 31, and it is possible to prevent the gasket 31 that has been subjected to the sterilization treatment from being contaminated.

In addition, the locking portion 56 is provided at the distal end portion on an outer peripheral surface of the cylindrical body 52 as illustrated in Fig. 5. The locking portion 56 protrudes outward from the outer peripheral surface of the cylindrical body 52. As illustrated in Figs. 1 and 3, the locking portion 56 is locked by the locking hole 47 of the syringe holder 13 when the cylindrical body 52 is inserted into the cylindrical hole of the barrel body portion 41 of the syringe holder 13.

Thus, the temporary holding portion 51 is opposed to the flange portion 24 of the syringe 11 as illustrated in Figs. 2 and 3. Further, the plunger member 12 is temporarily held by the temporary holding portion 51 in a state where the gasket 31 is opposed to the opening 24a of the syringe 11 with a gap between the gasket 31 and the opening 24a.

### 2. Manufacturing Method and Use Method of Drug-solution administration device

Next, a manufacturing method and a use method of the drug-solution administration device 1 having the above-described configuration will be described with reference to Figs. 1 to 10.

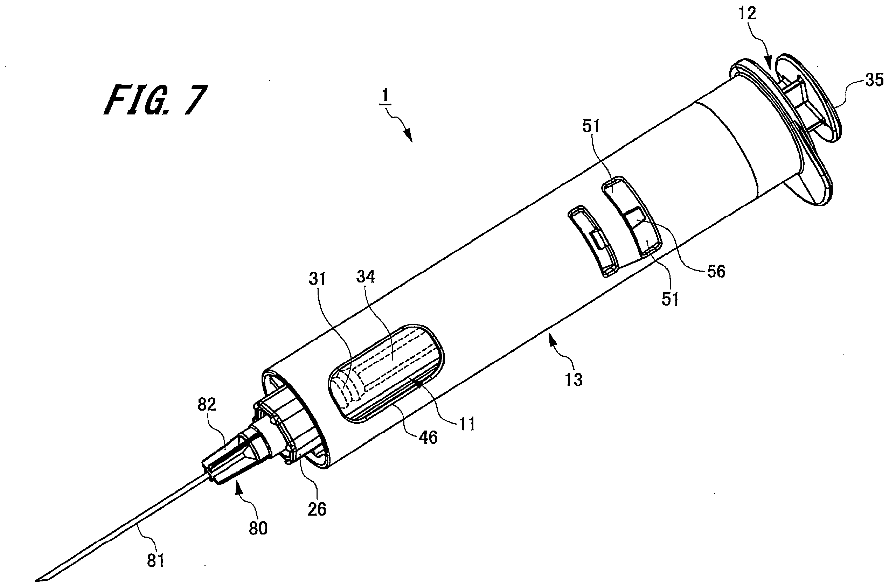

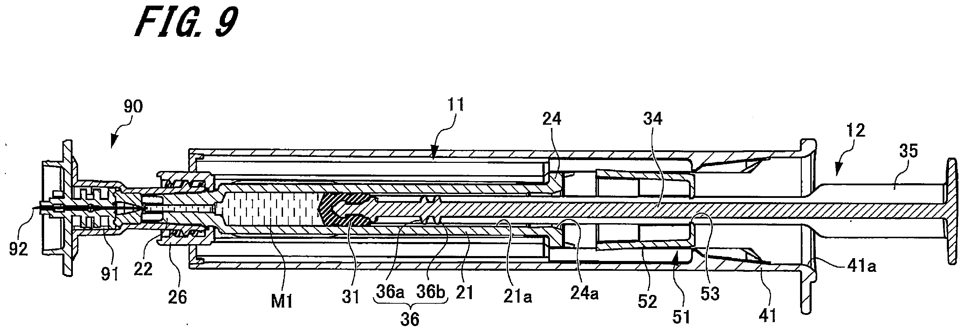

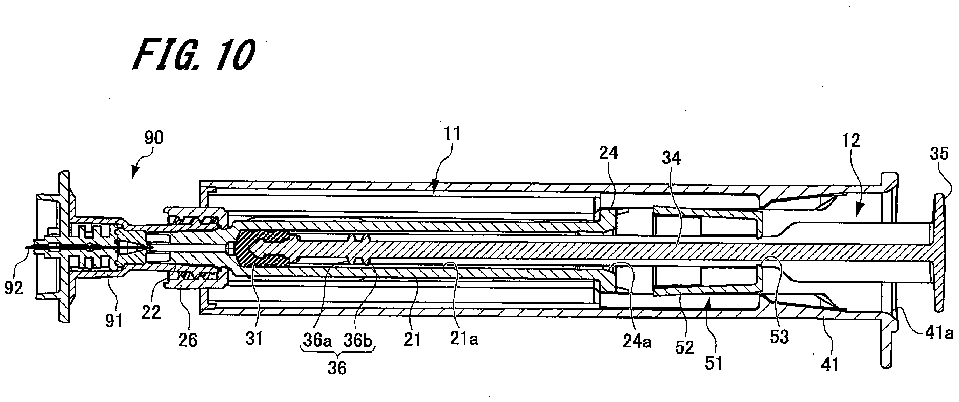

Fig. 7 is a perspective view illustrating the drug-solution administration device 1 at the time of sucking the drug solution, and Fig. 8 is a cross-sectional view illustrating the drug-solution administration device 1 after the sterilization treatment. Fig. 9 is a cross-sectional view illustrating a state after sucking the drug solution, and Fig. 10 is a cross-sectional view illustrating a state after administering the drug solution.

First, the syringe 11 is attached to the syringe holder 13 as illustrated in Figs. 1 and 2. Next, the plunger body 34 is inserted in the insertion hole formed at the distal end portions of the two holding-portion-side engaging portions 53 and 53 and the two holding-portion-side retaining portions 54 and 54 in the temporary holding portion 51 as illustrated in Fig. 6. Then, the plunger-side engaging portion 36 is engaged with the holding-portion-side engaging portion 53 of the temporary holding portion 51. In addition, the gasket 31 is connected to the connecting portion 34a (see Fig. 2) of the plunger body 34. As a result, the gasket 31 is covered by the cylindrical body 52 of the temporary holding portion 51, and the plunger member 12 is temporarily held by the temporary holding portion 51.

Next, the temporary holding portion 51 and the plunger member 12 are inserted into the cylindrical hole of the barrel body portion 41 of the syringe holder 13. Then, the locking portion 56 of the temporary holding portion 51 is locked by the locking hole 47 of the syringe holder 13 as illustrated in Figs. 1 and 3. As a result, the plunger member 12 is temporarily held by the temporary holding portion 51 in a state where the gasket 31 is opposed to the opening 24a of the syringe 11.

Next, the sterilization treatment is performed using high-temperature steam or gases such as high-pressure steam sterilization (autoclave) and ethylene oxide gas (EOG) sterilization. Here, the gasket 31 is not inserted into the cylindrical hole 21a of the syringe 11, and is arranged outside the syringe 11. Thus, a space between the two peak portions 32 of the gasket 31 is opened. In addition, a plurality of gaps is formed in the cylindrical body 52 of the temporary holding portion 51 at the distal end portion in the axial direction, between the holding-portion-side engaging portion 53 and the support rib 55, between the support rib 55 and the holding-portion-side retaining portion 54, and the like.

As a result, it is possible to cause the steam or gases to spread up to the gasket 31 attached to the plunger member 12 temporarily held by the temporary holding portion 51 and to the inside of the cylindrical hole 21a of the syringe 11. As a result, it is possible to reliably sterilize the gasket 31 and the syringe 11 as the whole in the state of being assembled in advance as the drug-solution administration device 1. With the above-described processing, the manufacturing method of the drug-solution administration device 1 is completed. Incidentally, the drug-solution administration device 1 may be wrapped in a state where the gasket 31 is opposed to the opening 24a of the syringe 11, or the drug-solution administration device 1 may be wrapped after inserting the gasket 31 into the cylindrical hole 21a of the syringe 11 to be described later.

When the sterilization treatment is completed, the plunger member 12 is operated to insert the gasket 31 into the cylindrical hole 21a of the syringe 11 at the time of wrapping the drug-solution administration device 1 or at the time of using the drug-solution administration device 1 as illustrated in Figs. 7 and 8.

The example illustrated in Fig. 7 illustrates an example in which an injection needle assembly 80 having a suction needle tube 81 configured to suck a drug solution from a vial containing the drug solution is attached to the syringe 11 in order to fill the liquid chamber of the syringe 11 with the drug solution. The needle hub 82 of the injection needle assembly 80 is screwed into the luer lock portion 26 of the syringe 11. As a result, the cylindrical hole 21a of the syringe 11 and the suction needle tube 81 of the injection needle assembly 80 communicate with each other via the discharge portion 22.

Incidentally, the injection needle assembly 80 illustrated in Fig. 7 may be attached to the syringe 11 after inserting the gasket 31 into the cylindrical hole 21a of the syringe 11 as illustrated in Fig. 8. In addition, the gasket 31 may be inserted into the cylindrical hole 21a of the syringe 11 after the injection needle assembly 80 illustrated in Fig. 7 is attached to the syringe 11.

When the plunger member 12 is pushed into the distal end portion of the syringe 11 as illustrated in Figs. 7 and 8, the engagement between the plunger-side engaging portion 36 of the plunger member 12 and the holding-portion-side engaging portion 53 of the temporary holding portion 51 is released. In addition, the plurality of support ribs 55 of the temporary holding portion 51 contact the plunger pieces 34b of the plunger body 34 as illustrated in Fig. 6. Thus, the plunger body 34 does not deviates from the opening 24a of the syringe 11, and the plunger body 34 and the gasket 31 can be inserted straight into the cylindrical hole 21a of the syringe 11.

In addition, when the gasket 31 and the plunger body 34 are inserted into the cylindrical hole 21a of the syringe 11, the plunger-side retaining portion 38 climbs over the holding-portion-side retaining portion 54 to move toward the distal end side of the holding-portion-side retaining portion 54. Then, the plunger-side retaining portion 38 can abut on the holding-portion-side retaining portion 54 from the distal end portion side. As a result, it is possible to prevent the plunger member 12 from falling out of the temporary holding portion 51 that has been attached to the syringe holder 13. At this time, a distal end surface of the gasket 31 is spaced apart from the distal end surface of the cylindrical hole 22a of the body portion 21.

The suction needle tube 81 of the injection needle assembly 80 pierces through the vial, and the plunger member 12 is pulled toward the proximal end of the syringe 11. As a result, the drug solution M1 is sucked through the suction needle tube 81 of the injection needle assembly 80 so that the liquid chamber of the syringe 11 is filled with the drug solution M1. When the filling of the drug solution M1 into the liquid chamber of the syringe 11 is completed, the injection needle assembly 80 is removed from the luer lock portion 26.

Then, the injection needle assembly 90 having an administration needle tube 91 for piercing the living body is attached to the syringe 11 as illustrated in Fig. 9. That is, the needle hub 92 of the injection needle assembly 90 is screwed into the luer lock portion 26 of the syringe 11 as illustrated in Fig. 9. As a result, the administration needle tube 91 of the injection needle assembly 90 communicates with the cylindrical hole 21a of the syringe 11 via the discharge portion 22.

Next, the administration needle tube 91 pierces through the user's skin. Then, the gasket 31 is slidably moved toward the distal end portion of the syringe 11 by operating the plunger member 12. As a result, the drug solution M1 filling the liquid chamber of the syringe 11 is pushed out of the administration needle tube 91 by the gasket 31. As a result, the administration of the drug solution to the living body using the drug-solution administration device 1 is completed.

As above, the embodiment of the present invention has been described including the operational effects thereof. However, the drug-solution administration device of the present invention is not limited to the above-described embodiment, and various modifications can be made within a scope not departing from the invention described in the claims.

Incidentally, various other administration parts such as a needle-free syringe having no needle tube and an intranasal administration device for administering a drug solution to a nasal cavity or the like can be applied as an administration part to be attached to the drug-solution administration device 1 as long as the part is attached by screwing.

In addition, the example in which the luer lock portion 26 is provided as the screw portion has been described in the above-described embodiment, but the present invention is not limited thereto. A male screw portion may be provided in the discharge portion 22 to be screwed with a female screw portion provided in the administrate part.

Further, the example in which the syringe 11 and the temporary holding portion 51 are attached to the syringe holder 13 has been described in the above-described embodiment, but the present invention is not limited thereto. For example, the temporary holding portion 51 and the syringe holder 13 may be integrally molded, or the syringe holder 13 is not necessarily provided. When the syringe holder 13 is not provided, the temporary holding portion may be releasably attached to the flange portion or the like of the syringe, or the temporary holding portion may be fixed to the syringe by adhesion or welding. Alternatively, the temporary holding portion and the syringe may be integrally molded.

As described above, the gasket 31 is covered by the cylindrical body 52 of the temporary holding portion 51 until the insertion of the gasket 31 into the syringe 11 after the sterilization treatment. Thus, even when the syringe holder 13 is not provided, it is possible to prevent the finger of the user from contacting the gasket 31.

Further, the example in which the plunger member 12 is temporarily held by the temporary holding portion 51 by engaging the plunger-side engaging portion 36 and the holding-portion-side engaging portion 53 has been described, but the present invention is not limited thereto. For example, the plunger body of the plunger member and the cylinder body of the temporary holding portion may be fixed by ultrasonic welding or laser welding so as to temporarily hold the plunger member by the temporary holding portion. Incidentally, a fixing strength between the plunger body and the cylindrical body is set to a strength at which the plunger body and the cylindrical body are broken when the plunger member is pressed after the sterilization treatment. Alternatively, the temporary holding portion and the plunger member may be integrally molded by providing a thin portion which is broken when the plunger member is pressed.

### Reference Signs List

- 1: drug-solution administration device
- 11: syringe
- 12: plunger member
- 13: syringe holder
- 21: body portion
- 21a: cylindrical hole
- 22: discharge portion
- 22a: cylindrical hole
- 24: flange portion
- 24a: opening
- 26: luer lock portion
- 31: gasket
- 31a: connecting hole
- 32: peak portion
- 34: plunger body
- 34a: connecting portion
- 34b: plunger piece
- 35: operation portion
- 36: plunger-side engaging portion
- 36a: first engaging portion
- 36b: second engaging portion
- 38: plunger-side retaining portion
- 41: barrel body portion
- 41a: holder opening
- 42: through-hole
- 46: viewing window
- 47: locking hole
- 48: holder collar
- 51: temporary holding portion
- 52: barrel body
- 53: holding-portion-side engaging portion
- 54: holding-portion-side retaining portion (retaining portion)
- 55: support rib (support portion)
- 56: locking portion
- 80, 90: injection needle assembly
- 81: suction needle tube
- 82, 92: needle hub
- 91: administration needle tube

## Claims

1. A drug-solution administration device (1) comprising:
a syringe (11) that includes a cylindrical body portion (21) configured to be filled with a drug solution therein, a discharge portion (22) formed at a distal end portion of the body portion (21), and an opening (24a) formed at a proximal end portion of the body portion (21);
a gasket (31) slidable inside a cylindrical hole (21a) of the body portion (21);
a plunger member (12) including a plunger body (34) to which the gasket (31) is attached;
a syringe holder (13) for holding the syringe (11); and
a temporary holding portion (51) provided in the syringe holder (13),
**characterized in that**
the temporary holding portion (51) includes a locking portion (56) configured to be locked by a locking hole (47) of the syringe holder (13);
a holding-portion-side engaging portion (53) releasably engaged with a plunger-side engaging portion (36) of the plunger body (34) such that the plunger member (12) is releasably held by the temporary holding portion (51) by engagement between the plunger-side engaging portion (36) and the holding-portion-side engaging portion (53) in a state in which the opening (24a) of the body portion (21) and a distal end surface of the gasket (31) are opposed to each other with a gap therebetween.

2. A drug-solution administration device (1) according to claim 1 wherein
an injection needle assembly (90), which comprises a needle tube including a needle tip that is configured to pierce a living body, or another injection needle assembly (80), which comprises a suction needle tube (81) including a needle tip that is configured to suck the drug solution, are detachably mounted to the discharge portion (22) of the syringe (11).

3. The drug-solution administration device (1) according to claim 1 or 2, wherein holding of the plunger member (12) by the temporary holding portion (51) is releasable as the plunger member (12) is pushed toward a distal end of the syringe (11).

4. The drug-solution administration device (1) according to claim 1, wherein the plunger-side engaging portion (36) comprises a first engaging portion (36a) engaged with the holding-portion-side engaging portion (53) from a distal end side and a second engaging portion (36b) engaged with the holding-portion-side engaging portion (53) from a proximal end side, and wherein the plunger member (12) is releasably held by the temporary holding portion (51) as the holding-portion-side engaging portion (53) is positioned between the first engaging portion (36a) and the second engaging portion (36b).

5. The drug-solution administration device (1) according to claim 4, wherein the plunger-side engaging portions (36a, 36b) are provided at two places that are positioned to be opposed to each other with an axial center of the plunger body (34) interposed therebetween, and wherein the holding-portion-side engaging portion (53) is provided at two places that are positioned to be opposed to each other with an axial center of the body portion (21) interposed therebetween.

6. The drug-solution administration device (1) according to any one of claims 4 to 5, wherein the plunger body (34) comprises a plunger-side retaining portion (38) provided at a position proximal of the plunger-side engaging portion (36) and different from the plunger-side engaging portion (36) around the axial center of the plunger body (34), wherein drug-solution administration device (1) further comprises a retaining portion that is provided at a position different from the holding-portion-side engaging portion (53) around the axial center of the body portion (21) and that is configured to abut on a plunger-side retaining portion (38) from a proximal end side, wherein the plunger-side retaining portion (38) and a retaining portion (54) are configured to restrict a movement of the plunger body (34) toward the proximal end side when the plunger-side retaining portion (38) climbs over the retaining portion (54) from the proximal end side and the plunger-side retaining portion (38) and the retaining portion (54) abut on each other, and wherein the distal end surface of the gasket (31) is spaced apart from a distal end surface of the cylindrical hole (21a, 22a) in the body portion (21) when the plunger-side retaining portion (38) and the retaining portion(54) abut on each other.

7. The drug-solution administration device according to any one of claims 1 to 6, wherein the temporary holding portion (51) comprises a support portion (55) that supports the plunger body (34), and wherein an inclination of an axis of the plunger body (34) with respect to an axis of the body portion (21) is restricted by the support portion.

8. A method of manufacturing a drug-solution administration device (1) according to any of claims 1 to 7,
the method comprising:
a step of attaching the syringe (11) to the syringe holder (13),
a step of engaging the plunger-side engaging portion (36) with the holding-portion-side engaging portion (53) of the temporary holding portion (51), a step of attaching the gasket (31) to the plunger body (34) of the plunger member (12),
a step of inserting the temporary holding portion (51) and the plunger member (12) into a cylindrical hole of a barrel body portion (41) of the syringe holder (1),
a step of locking the locking portion (56) of the temporary holding portion (51) by the locking hole (47) of the syringe holder (13)
a step of holding the plunger member (12) by the temporary holding portion (51) in a state in which the opening (24a) formed in the proximal end portion of the body portion (21) of the syringe (11) and the distal end surface of the gasket (31) are opposed to each other with the gap therebetween; and
a step of sterilizing the syringe (11), the gasket (31), the plunger member (12), and the temporary holding portion (51) with a sterilization gas or high-pressure steam in a state in which the plunger member (12) is held by the temporary holding portion (51).

## Patentansprüche

1. Vorrichtung (1) zur Verabreichung einer Arzneimittellösung, umfassend:
eine Spritze (11), die einen zylindrischen Körperabschnitt (21), der so konfiguriert ist, dass er darin mit einer Arzneimittellösung gefüllt werden kann, einen Auslassabschnitt (22), der an einem distalen Endabschnitt des Körperabschnitts (21) ausgebildet ist, und eine Öffnung (24a), die an einem proximalen Endabschnitt des Körperabschnitts (21) ausgebildet ist, umfasst;
eine Dichtung (31), die im Inneren eines zylindrischen Loches (21a) des Körperabschnitts (21) verschiebbar ist;
ein Kolbenelement (12), das einen Kolbenkörper (34) umfasst, an dem die Dichtung (31) angebracht ist;
einen Spritzenhalterung (13) zum Halten der Spritze (11); und
einen vorübergehenden Halteabschnitt (51), der in der Spritzenhalterung (13) vorgesehen ist,
**dadurch gekennzeichnet, dass**
der vorübergehende Halteabschnitt (51) einen Verriegelungsabschnitt (56) umfasst, der so konfiguriert ist, dass er durch ein Verriegelungsloch (47) der Spritzenhalterung (13) verriegelt wird;
ein auf der Seite des Halteabschnitts liegender Eingriffsabschnitt (53), der lösbar mit einem auf der Seite des Kolbens liegenden Eingriffsabschnitt (36) des Kolbenkörpers (34) derart in Eingriff steht, dass das Kolbenelement (12) lösbar durch den vorübergehenden Halteabschnitt (51) durch den Eingriff zwischen dem auf der Seite des Kolbens liegenden Eingriffsabschnitt (36) und dem auf der Seite des Halteabschnitts liegenden Eingriffsabschnitt (53) in einem Zustand lösbar gehalten wird, in welchem die Öffnung (24a) des Körperabschnitts (21) und eine distale Endfläche der Dichtung (31) einander mit einem Spalt dort dazwischen gegenüberliegen.

2. Vorrichtung (1) zur Verabreichung einer Arzneimittellösung nach Anspruch 1, wobei eine Injektionsnadel-Anordnung (90), die ein Nadelrohr umfasst, das eine Nadelspitze aufweist, die so konfiguriert ist, dass sie in einen lebenden Körper einsticht, oder eine andere Injektionsnadel-Anordnung (80), die ein Saugnadelrohr (81) umfasst, das eine Nadelspitze aufweist, die so konfiguriert ist, dass sie die Arzneimittellösung ansaugt, abnehmbar an dem Auslassabschnitt (22) der Spritze (11) angebracht sind.

3. Vorrichtung (1) zur Verabreichung einer Arzneimittellösung nach Anspruch 1 oder 2, wobei das Halten des Kolbenelements (12) durch den vorübergehenden Halteabschnitt (51) lösbar ist, wenn das Kolbenelement (12) in Richtung eines distalen Endes der Spritze (11) gedrückt wird.

4. Vorrichtung (1) zur Verabreichung einer Arzneimittellösung nach Anspruch 1, wobei der auf der Seite des Kolbens liegende Eingriffsabschnitt (36) einen ersten Eingriffsabschnitt (36a), der von einer distalen Endseite her mit dem auf der Seite des Halteabschnitts liegenden Eingriffsabschnitt (53) in Eingriff steht, und einen zweiten Eingriffsabschnitt (36b) umfasst, der von einer proximalen Endseite her mit dem auf der Seite des Halteabschnitts liegenden Eingriffsabschnitt (53) in Eingriff steht, und wobei das Kolbenelement (12) durch den vorübergehenden Halteabschnitt (51) lösbar gehalten wird, wenn der auf der Seite des Halteabschnitts liegende Eingriffsabschnitt (53) zwischen dem ersten Eingriffsabschnitt (36a) und dem zweiten Eingriffsabschnitt (36b) positioniert ist.

5. Vorrichtung (1) zur Verabreichung einer Arzneimittellösung nach Anspruch 4, wobei die auf der Seite des Kolbens liegenden Eingriffsabschnitte (36a, 36b) an zwei Stellen vorgesehen sind, die so positioniert sind, dass sie einander gegenüberliegen, wobei eine axiale Mitte des Kolbenkörpers (34) dazwischen angeordnet ist, und wobei der auf der Seite des Halteabschnitts liegende Eingriffsabschnitt (53) an zwei Stellen vorgesehen ist, die so positioniert sind, dass sie einander gegenüberliegen, wobei eine axiale Mitte des Körperabschnitts (21) dazwischen angeordnet ist.

6. Vorrichtung (1) zur Verabreichung einer Arzneimittellösung nach einem der Ansprüche 4 bis 5, wobei der Kolbenkörper (34) einen auf der Seite des Kolbens liegenden Rückhalteabschnitt (38) aufweist, der an einer Position proximal des auf der Seite des Kolbens liegenden Eingriffsabschnitts (36) vorgesehen ist und sich von dem auf der Seite des Kolbens liegenden Eingriffsabschnitt (36) rund um die axiale Mitte des Kolbenkörpers (34) unterscheidet, wobei die Vorrichtung (1) zur Verabreichung einer Arzneimittellösung ferner einen Rückhalteabschnitt umfasst, der an einer Position vorgesehen ist, die sich von dem auf der Seite des Halteabschnitts liegenden Eingriffsabschnitt (53) rund um die axiale Mitte des Körperabschnitts (21) unterscheidet, und der so konfiguriert ist, dass er an einem auf der Seite des Kolbens liegenden Rückhalteabschnitt (38) von einer proximalen Endseite anliegt, wobei der auf der Seite des Kolbens liegende Rückhalteabschnitt (38) und ein Rückhalteabschnitt (54) so konfiguriert sind, dass sie eine Bewegung des Kolbenkörpers (34) in Richtung der proximalen Endseite einschränken, wenn der auf der Seite des Kolbens liegende Rückhalteabschnitt (38) über den Rückhalteabschnitt (54) von der proximalen Endseite klettert und der auf der Seite des Kolbens liegende Rückhalteabschnitt (38) und der Rückhalteabschnitt (54) aneinander anliegen, und wobei die distale Endfläche der Dichtung (31) von einer distalen Endfläche des zylindrischen Loches (21a, 22a) in dem Körperabschnitt (21) beabstandet ist, wenn der auf der Seite des Kolbens liegende Rückhalteabschnitt (38) und der Rückhalteabschnitt (54) aneinander anliegen.

7. Vorrichtung zur Verabreichung einer Arzneimittellösung nach einem der Ansprüche 1 bis 6, wobei der vorübergehende Halteabschnitt (51) einen Stützabschnitt (55) umfasst, der den Kolbenkörper (34) stützt, und wobei eine Neigung einer Achse des Kolbenkörpers (34) in Bezug auf eine Achse des Körperabschnitts (21) durch den Stützabschnitt begrenzt ist.

8. Verfahren zur Herstellung einer Vorrichtung (1) zur Verabreichung einer Arzneimittellösung nach einem der Ansprüche 1 bis 7,
wobei das Verfahren umfasst:
einen Schritt des Anbringens der Spritze (11) an der Spritzenhalterung (13),
einen Schritt des Eingreifens des auf der Seite des Kolbens liegenden Eingriffsabschnitts (36) mit dem auf der Seite des Halterungsabschnitts liegenden Eingriffsabschnitt (53) des temporären Halteabschnitts (51),
einen Schritt des Anbringens der Dichtung (31) an dem Kolbenkörper (34) des Kolbenelements (12),
einen Schritt des Einsetzens des temporären Halteabschnitts (51) und des Kolbenelements (12) in ein zylindrisches Loch eines Zylinderkörperabschnitts (41) der Spritzenhalterung (1),
einen Schritt des Verriegelns des Verriegelungsabschnitts (56) des temporären Halteabschnitts (51) durch das Verriegelungsloch (47) der Spritzenhalterung (13),
einen Schritt des Haltens des Kolbenelements (12) durch den temporären Halteabschnitt (51) in einem Zustand, in dem die Öffnung (24a), die in dem proximalen Endabschnitt des Körperabschnitts (21) der Spritze (11) ausgebildet ist, und die distale Endfläche der Dichtung (31) einander mit dem dazwischen liegenden Spalt gegenüberliegen;
und
einen Schritt des Sterilisierens der Spritze (11), der Dichtung (31), des Kolbenelements (12) und des temporären Halteabschnitts (51) mit einem Sterilisationsgas oder Hochdruckdampf in einem Zustand, in dem das Kolbenelement (12) durch den temporären Halteabschnitt (51) gehalten wird.

## Revendications

1. Dispositif d'administration de solution médicamenteuse (1) comprenant :
une seringue (11) qui comprend une partie cylindrique de corps (21) configurée pour être remplie avec une solution médicamenteuse, une partie de décharge (22) formée au niveau d'une partie d'extrémité distale de la partie de corps (21),
et une ouverture (24a) formée au niveau d'une partie d'extrémité proximale de la partie de corps (21) ;
un joint (31) coulissant à l'intérieur d'un trou cylindrique (21a) de la partie de corps (21) ;
un élément de piston (12) comprenant un corps de piston (34) auquel le joint (31) est fixé ;
un support de seringue (13) permettant de maintenir la seringue (11) ; et
une partie de maintien temporaire (51) prévue dans le support de seringue (13),
**caractérisé en ce que**
la partie de maintien temporaire (51) comprend une partie de verrouillage (56) configurée pour être verrouillée par un trou de verrouillage (47) du support de seringue (13) ;
une partie d'engagement côté partie de maintien (53) mise en prise de manière amovible avec une partie d'engagement côté piston (36) du corps de piston (34) de telle sorte que l'élément de piston (12) est maintenu de manière amovible par la partie de maintien temporaire (51) par engagement entre la partie d'engagement côté piston (36) et la partie d'engagement côté partie de maintien (53) dans un état dans lequel l'ouverture (24a) de la partie de corps (21) et une surface d'extrémité distale du joint (31) sont opposées l'une à l'autre avec un espace entre elles.

2. Dispositif d'administration de solution médicamenteuse (1) selon la revendication 1, dans lequel un ensemble d'aiguille d'injection (90), qui comprend un tube d'aiguille comprenant une pointe d'aiguille qui est configurée pour percer un corps vivant, ou un autre ensemble d'aiguille d'injection (80), qui comprend un tube d'aiguille d'aspiration (81) comprenant une pointe d'aiguille qui est configurée pour aspirer la solution médicamenteuse, sont montés de manière amovible sur la partie de décharge (22) de la seringue (11).

3. Dispositif d'administration de solution médicamenteuse (1) selon la revendication 1 ou la revendication 2, dans lequel la partie de maintien temporaire (51) de l'élément de piston (12) peut être libérée lorsque l'élément de piston (12) est poussé vers l'extrémité distale de la seringue (11).

4. Dispositif d'administration de solution médicamenteuse (1) selon la revendication 1, dans lequel la partie d'engagement côté piston (36) comprend une première partie d'engagement (36a) en prise avec la partie d'engagement côté partie de maintien (53) à partir d'un côté d'extrémité distale et une deuxième partie d'engagement (36b) en prise avec la partie d'engagement côté partie de maintien (53) à partir d'un côté d'extrémité proximale, et dans lequel l'élément de piston (12) est maintenu de manière amovible au moyen de la partie de maintien temporaire (51) lorsque la partie d'engagement côté partie de maintien (53) est positionnée entre la première partie d'engagement (36a) et la deuxième partie d'engagement (36b).

5. Dispositif d'administration de solution médicamenteuse (1) selon la revendication 4, dans lequel les parties d'engagement côté piston (36a, 36b) sont prévues à deux endroits qui sont positionnés pour être opposés l'un à l'autre avec un centre axial du corps de piston (34) interposé entre eux, et dans lequel la partie d'engagement côté partie de maintien (53) est prévue à deux endroits qui sont positionnés pour être opposés l'un à l'autre avec un centre axial de la partie de corps (21) interposé entre eux.

6. Dispositif d'administration de solution médicamenteuse (1) selon l'une quelconque des revendications 4 à 5, dans lequel le corps de piston (34) comprend une partie de retenue côté piston (38) prévue à une position proximale de la partie d'engagement côté piston (36) et différente de la partie d'engagement côté piston (36) autour du centre axial du corps de piston (34), dans lequel le dispositif d'administration de solution médicamenteuse (1) comprend en outre une partie de retenue qui est prévue à une position différente de la partie d'engagement côté partie de maintien (53) autour du centre axial de la partie de corps (21) et qui est configurée pour venir en butée sur une partie de retenue côté piston (38) à partir d'un côté d'extrémité proximale, dans lequel la partie de retenue côté piston (38) et une partie de retenue (54) sont configurées pour restreindre un mouvement du corps de piston (34) en direction du côté de l'extrémité proximale lorsque la partie de retenue côté piston (38) grimpe sur la partie de retenue (54) depuis le côté de l'extrémité proximale et la partie de retenue côté piston (38) et la partie de retenue (54) viennent en butée l'une contre l'autre, et dans lequel la surface d'extrémité distale du joint (31) est séparée de la surface d'extrémité distale du trou cylindrique (21a, 22a) dans la partie du corps (21) lorsque la partie de retenue côté piston (38) et la partie de retenue (54) viennent en butée l'une contre l'autre.

7. Dispositif d'administration de solution médicamenteuse selon l'une quelconque des revendications 1 à 6, dans lequel la partie de maintien temporaire (51) comprend une partie de support (55) qui supporte le corps du piston (34), et dans lequel une inclinaison d'un axe du corps de piston (34) par rapport à un axe de la partie de corps (21) est limitée par la partie de support.

8. Procédé de fabrication d'un dispositif d'administration de solution médicamenteuse (1) selon l'une quelconque des revendications 1 à 7,
le procédé comprenant :
une étape de fixation de la seringue (11) au support de seringue (13),
une étape d'engagement de la partie d'engagement côté piston (36) avec la partie d'engagement côté partie de maintien (53) de la partie de maintien temporaire (51),
une étape de fixation du joint (31) au corps de piston (34) de l'élément de piston (12),
une étape d'insertion de la partie de maintien temporaire (51) et de l'élément de piston (12) dans un trou cylindrique d'une partie de corps de barillet (41) du support de seringue (1),
une étape de verrouillage de la partie de verrouillage (56) de la partie de maintien temporaire (51) au moyen du trou de verrouillage (47) du support de seringue (13),
une étape de maintien de l'élément de piston (12) au moyen de la partie de maintien temporaire (51) dans un état dans lequel l'ouverture (24a) formée dans la partie d'extrémité proximale de la partie de corps (21) de la seringue (11) et la surface d'extrémité distale du joint (31) sont opposées l'une à l'autre avec l'espace entre elles ;
et une étape de stérilisation de la seringue (11), du joint (31), de l'élément de piston (12) et de la partie de maintien temporaire (51) avec un gaz de stérilisation ou de la vapeur à haute pression dans un état dans lequel l'élément de piston (12) est maintenu par la partie de maintien temporaire (51).
